# EUROPEAN PATENT SPECIFICATION

(11) **EP 1 184 012 B1**
(45) Date of publication and mention of the grant of the patent: **21.12.2005**
(21) Application number: 01116733.5
(22) Date of filing: 18.07.2001
(51) Int. Cl.: A61F 13/15

(54) **Shorts type disposable diaper**
Wegwerfwindel in Höschenform
Couche jetable de type culotte

(30) Priority: 04.09.2000 JP 2000266659
(43) Date of publication of application: 06.03.2002
(73) Proprietor: KAO CORPORATION, Chuo-ku, Tokyo (JP)
(72) Inventor: Hayase, Toru, Ichikai-machi, Hagagun, Tochigi (JP); Murai, Atsushi, Ichikai-machi, Hagagun, Tochigi (JP); Sasaki, Jun, Ichikai-machi, Hagagun, Tochigi (JP)
(74) Representative: Vossius & Partner

(56) References cited:
- EP-A- 0 320 991
- EP-A- 0 901 780
- EP-A- 0 923 920

## Description

### BACKGROUND OF THE INVENTION

The present invention relates to a shorts type disposable diaper of which the liquid-absorbing area of the absorbent core can be made effective use of.

The assignee of this application has previously disclosed in United States Patents 5,415,649 and 5,449,353 a shorts type disposable diaper having a waist opening portion and a pair of leg opening portions in which gathers are formed separately and circumferentially around an entirety of each of the waist opening portion and the leg opening portions by providing elastic members at the periphery of the respective opening portions. The diaper also has gathers at a body-surrounding portion which is located between the waist opening portion and the leg opening portion. The gathers extend in the circumferential direction of the diaper and are formed by providing elastic members at the body-surrounding portion. The elastic members are provided across the absorbent core, or alternatively not provided on the absorbent core but provided in such a manner that the gathers are formed at the side portions of the body-surrounding portion.

Japanese Patent Laid-Open No. 2-4364 discloses a shorts type disposable diaper which has a pair of contractible side panels in side portions of the diaper connecting a rear portion and a front portion and has an elastic member in a waist opening portion. The disclosed shorts type diaper, however, tends to cause gapping between a wearer's skin and the waist or leg openings of the diaper and usually shows low flexibility to fit a large range of sizes of wearers due to poor elongation stress characteristics of the side panels. Further, in order to prevent the diaper from sagging or drooping while worn, the side panels should have an increased stress in elongation and contraction, which not only gives a wearer a feeling of being pressed but needs no little force to widen the waist opening, tending to make it difficult for a diaperer to put the diaper on a wearer or for a wearer to put it on.

Japanese Patent 2602408 discloses a shorts type disposable diaper having elastic members disposed around the waist, wherein the elastic members each have a part in a non-stretched state and a part in a stretched state. The non-stretched part of the elastic member is formed by adhering the elastic member to a backsheet with an adhesive in its non-stretched state. However, an operation of adhering an elastic member in a non-stretched state to some parts of sheeting and in a stretched state to other parts needs a large amount of an adhesive, is liable to involve processing troubles, and increases in production cost. Moreover the adhesive makes the diaper stiff as a whole to impair the texture. Because the non-stretched part and the stretched part, which have conflicting properties, are made of the same elastic member, it is difficult to control the stretching characteristics. As a result, the diaper fails to maintain sufficient tension to hold to the wearer and easily sags and droops while worn, or the diaper exerts too much tension to uncomfortably press a wearer's body.

EP-A-0 901 780 relates to absorbent articles such as diapers, as well as a manufacturing method thereof. In one embodiment of the diaper described in this document, a pair of elastic leakage protective members are disposed in such a manner that they cover around half of the lower portion of the respective leg holes. In addition, a waist gather is disposed along the waist holes so as to provide this portion with elasticity.

It is an object of the present invention to provide a shorts type disposable diaper which flexibly fits a large range of wearer sizes, is easy to put on a wearer, exerts moderate tension to maintain a snug fit to a wearer, and has a neat appearance on a wearer.

### SUMMARY OF THE INVENTION

This object is achieved with the features of the claims. In particular, the object of the present invention is accomplished by a shorts type disposable diaper comprising a liquid-permeable topsheet, a liquid-impermeable anti-leakage sheet and a liquid-retentive absorbent core, and having a plurality of body-surrounding elastic members provided at a body-surrounding portion which is located between a waist opening portion and a leg opening portion, said body-surrounding elastic members extending in the circumferential direction of the diaper, wherein:
said body-surrounding elastic members are fixedly disposed in at least side portions which extend outward from longitudinal side edges of said absorbent core in a stretched state so as to manifest elastic contractibility, but are not disposed in at least the center of the portion where said absorbent core exists, or alternatively, are disposed in the portion where said absorbent core exists in such a manner that elastic contractibility does not manifest in at least said center,
the ratio of the width of said absorbent core at the position which corresponds to the position where said body-surrounding elastic members are disposed to the width of said diaper is 30 to 60%, and
the ratio of the width of parts in which said elastic members manifest elastic contractibility to the width of said diaper is 40 to 95%.

### BRIEF DESCRIPTION OF THE DRAWINGS

The present invention will be more particularly described with reference to the accompanying drawings, in which:
Fig. 1 is a perspective view of an embodiment of the shorts type disposable diaper according to the present invention; and
Fig. 2A is a plan view of the topsheet side of the shorts type disposable diaper of Fig. 1 in the flat-out stretched state before assembly, and Fig. 2B is a cross-section of Fig. 2A along line b-b.

### DETAILED DESCRIPTION OF THE PREFERRED EMBODIMENTS

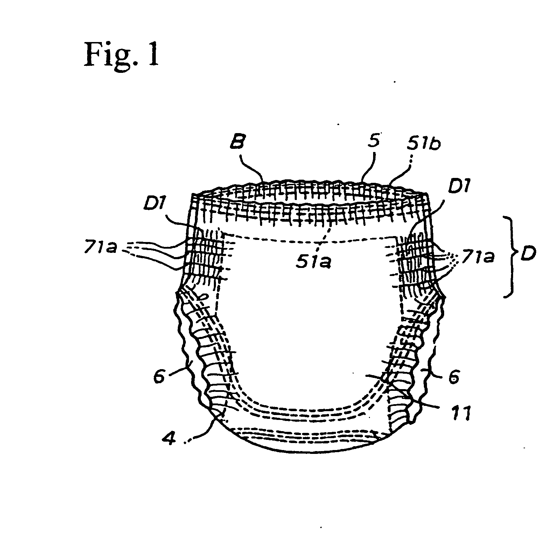

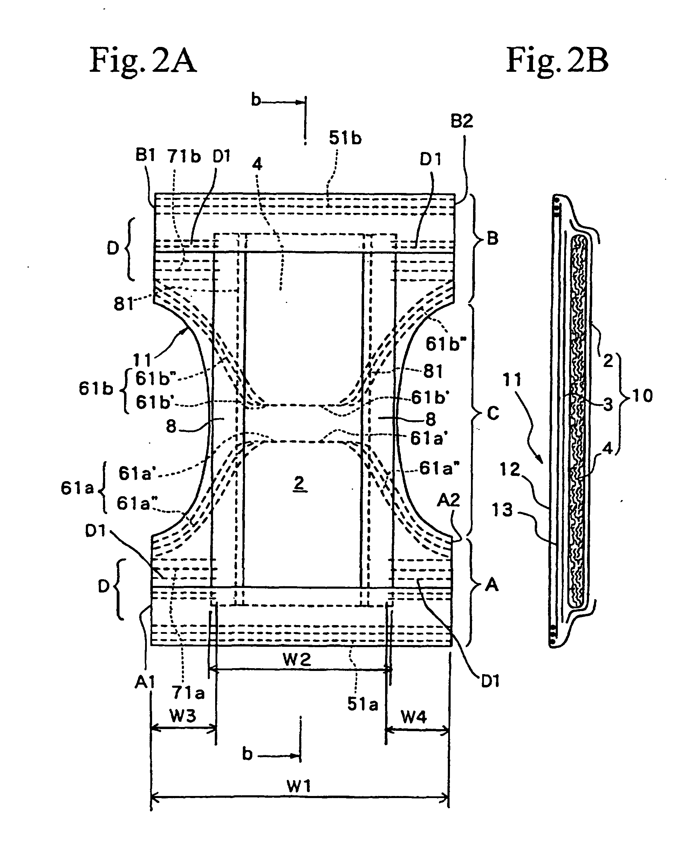

A preferred embodiment of the shorts type disposable diaper according to the present invention will be described with reference to the drawings. Fig. 1 is a perspective view of the diaper. Fig. 2A is a plan view of the diaper of Fig. 1 in its unfolded and stretched state before assembly, viewed from the topsheet side thereof. Fig. 2B is a cross-section of Fig. 2A along line b-b.

As shown in Figs. 1, 2A and 2B, the disposable diaper 1 of this embodiment is of shorts type and comprises an absorbent body 10 and an exterior member 11. The absorbent body 10 is substantially rectangular and comprises a liquid-permeable topsheet 2, a liquid-impermeable anti-leakage sheet 3 and a liquid retentive absorbent core 4 which is interposed between the topsheet 2 and the anti-leakage sheet 3. The exterior member 11 is disposed on the anti-leakage sheet 3 side of the absorbent body 10.

The diaper 1 is made up of a front portion A, which is disposed on the wearer's stomach side, a rear portion B, which is on the wearer's back side, and a crotch portion C, which is between the portions A and B. Side edges A1 and A2 of the front portion A and side edges B1 and B2 of the rear portion B are joined together, respectively, to form a waist opening portion 5 and a pair of leg opening portions 6. The joining is carried out by heat sealing, RF sealing, ultrasonic sealing, and the like.

The topsheet 2, the anti-leakage sheet 3, and the absorbent core 4 are joined together by a prescribed means to form a rectangular absorbent body 10. The topsheet 2, the anti-leakage sheet 3, and the absorbent core 4 are all rectangular. The topsheet 2 and the anti-leakage sheet 3 can be of those commonly employed in this type of diapers. The absorbent core 4 is made up of superabsorbent polymer particles and a fibrous material and covered by tissue (not shown).

As shown in Fig. 2A, a pair of cuffs (or flaps) 8 made of a liquid-resistant or liquid-impermeable and breathable material are provided on the lateral sides of the absorbent body 10. Each cuff 8 has its one longitudinal end fixed to the topsheet 2 over the length of the absorbent body 10 with the other end free. A cuff elastic member 81 is fixed in its stretched state to each cuff 8 in the vicinity of its free end, whereby the cuffs 8 stand upright to present barriers against the flow of liquid waste flowing in the lateral direction.

As shown in Fig. 2B, the absorbent body 10 is disposed on the central portion of the exterior member 11 with their longitudinal directions agreeing with each other. The absorbent body 10 and the exterior member 11 are fixed together by partial bonding. Such a way of fixing is more effective to prevent unfavorable gathering (described later) of the absorbent core 4.

As shown in Fig. 2B, the exterior member 11 comprises at least two sheets; an outer sheet 12 and an inner sheet 13 that is disposed on the inner side of the outer sheet 12. These two sheets are made of, for example, nonwoven fabric. The exterior member 11 forms the contour of the diaper 1, and the outer sheet 12 of the exterior member 11 serves as the outermost surface of the diaper 1. The inner sheet 13 is joined to the inner side of the outer sheet 12 with an adhesive, such as a hot-melt self-adhesive. The exterior member 11 has an almost rectangular shape with middle portion of each lateral side curved inward to make a hourglass shape.

The exterior member 11 extends outward from the front and rear edges of the absorbent body 10, and the extensions are folded back on the topsheet 2 side with the folded ends being positioned on the absorbent core 4 as shown in Fig. 2B.

As shown in Fig. 2A, a plurality of waist elastic members 51a and 51b are fixedly provided in their stretched state at the front and rear ends of the exterior member 11 between the outer sheet 12 and the inner sheet 13 over the whole width of the exterior member. The elastic members 51a and 51b are disposed such that their ends meet with an overlap when the side edges A1 and A2 of the front portion A and the side edges B 1 and B2 of the rear portion B are respectively joined together. Thus there are formed substantially continuous gathers encircling the waist opening portion 5 of the diaper 1.

As shown in Fig. 2A, the curved portions of the exterior member 11 (crotch portion C) are provided with leg elastic members 61a and 61b. Each of the elastic members 61a and 61b comprises a middle part 61a', 61b' and side parts 61a", 61b" which are extensions from both ends of the respective middle part. The middle parts 61a' and 61b' are disposed across the crotch portion C, and the side portions 61a" and 61b" are along the curves of the crotch portion C. The elastic members 61a and 61b are held between the outer sheet 12 and the inner sheet 13 with their side portions 61a" and 61b" being fixed in their stretched state by an appropriate means. When the side edges A1 and A2 of the front portion A and the side edges B1 and B2 of the rear portion B are joined together, respectively, the ends of the elastic member 61a and those of the elastic member 61b meet with an overlap to make a substantially continuous loop of gathers encircling the leg opening portions 6.

The waist elastic members 51a, 51b, the leg elastic members 61a, 61b, and the cuff elastic member 81 are preferably bands or strings made of natural rubber, polyurethane resins, foamed urethane resins, and the like. The waist elastic members 51a, 51b preferably have a 30% elongation stress of 98 to 980 mN (10 to 100 gf), particularly 147 to 785 mN (15 to 80 gf) and are preferably fixedly held between the outer sheet 12 and the inner sheet 13 in a stretched state at a elongation of 60 to 160%. The term " elongation (%)" refers to a percentage of an increase in length to the non-stretched state length. For example, when a 10 cm elastic member is stretched to 20 cm, the elongation is 100%.

As shown in Figs. 1, 2A and 2B, a plurality of body-surrounding elastic members 71a, 71b are provided at the body-surrounding portion D in the exterior member 11. The body-surrounding portion D is located between the front or rear end and the curved portion of the exterior member 11. The body-surrounding elastic members 71a, 71b extend in the width direction of the exterior member 11. It can be said that the body-surrounding portion D is also located between the waist opening portion 5 and the leg opening portion 6 of the diaper 1. The body-surrounding elastic members 71a, 71b are of string form and are disposed in portions D1 which are located in the front and rear potions A and B. The portions D1 are part of the body-surrounding portion D and extend outward from both longitudinal side edges of the absorbent core 4. The portion D1 will sometimes be referred to as "body-surrounding side portion". The body-surrounding elastic members 71a and 71b are each secured in their stretched state between the outer sheet 12 and the inner sheet 13 so as to manifest elastic contractibility. The body-surrounding elastic members 71a and 71b are each discontinuously joined to the outer sheet 12 and the inner sheet 13 by known bonding means such as an adhesive or heat-sealing. They are disposed such that their ends meet with an overlap when the side edges A1 and A2 of the front portion A and the side edges B1 and B2 of the rear portion B are respectively joined together as shown in Fig. 1. Thus, the body-surrounding elastic members 71a and 71b are disposed in the body-surrounding portion in such a manner that they extend in the circumferential direction of the diaper 1. As a result, gathers are formed in the region in the body-surrounding side portions D1 where the body-surrounding elastic members 71a and 71b are provided.

The body-surrounding elastic members 71a and 71b are not disposed in at least the center of the portion where the absorbent core 4 exists. Or alternatively, when the body-surrounding elastic members 71a and 71b are disposed in the portion where the absorbent core 4 exists, the body-surrounding elastic members 71a and 71b are disposed in such a manner that elastic contractibility does not manifest in at least the above center. In the particular embodiment, the body-surrounding elastic members 71a and 71b are disposed in the body-surrounding side portions D1 with their inward ends slightly overlapping the side edges of the absorbent core 4, and there is provided no elastic member in the area from the center in the width direction to the vicinity of both the lateral side edges of the absorbent core 4. The parts of the body-surrounding elastic members 71a and 71b that overlap the lateral side edges of the absorbent core 4 are in a non-stretched state to have no contractibility. As a result, gathers are not formed in the area of the diaper 1 where the absorbent core 4 is disposed.

In order to prevent bunching or gathering of the absorbent core 4, it is the most preferred that no body-surrounding elastic member be provided in the area where the absorbent core 4 exists or, if any be provided, they are provided in a non-contractible state on that area. Prevention of the absorbent core's bunching can be achieved in the practice only if the elastic members provided in the area corresponding to the absorbent core 4 have a non-contractible part. It is allowable for the contractible elastic members 71a and 71b to be present in the area corresponding to the absorbent core 4 in a ratio of preferably not more than 60%, more preferably not more than 40%, based on the width of the absorbent core 4.

As long as the body-surrounding elastic members 71a and 71b are arranged in the above-described configuration, the absorbent core 4 is prevented from bunching or gathering. As a result, the liquid absorbing area of the absorbent core 4 can be utilized effectively, and gapping formed between the diaper 1 and the wearer's body hardly occurs to effectively prevent leakage. It may be conceivable to make the inner dimensions of a diaper agree with the waistline of a wearer so as to prevent bunching of the absorbent core, and wherein the body-surrounding elastic members are extended in approximately a pre-elongated state. In such a case, however, the size of the diaper around the waist is so small that the diaper is difficult to put on a wearer. In addition, in such a diaper design, the body-surrounding elastic members may produce the maximum stress when the maximum dimensions of the diaper agree with the waistline of a wearer, which is most impracticable.

According to the above-described configuration of the invention, the absorbent core 4 does not suffer from contraction due to excessive bunching. Thus, even though the width or the thickness of the absorbent core 4 is made smaller than that of conventional disposable diapers, the diaper 1 successfully exhibits equality in absorbing performance to conventional diapers, which realizes reduction in material cost. More specifically, the ratio of the width W2 (see Fig. 2) of the absorbent core 4 measured in its flat state at the position which corresponds to the position where the body-surrounding elastic members 71a and 71b are disposed to the width W1 (see Fig. 2) of the diaper 1 measured in its flat state is 30 to 60%, preferably 40 to 50%, still preferably 43 to 48%. If the ratio W2/W1 is less than 30%, sufficient absorbing performance is not exerted. A W2/W1 ratio exceeding 60% has no merit from the standpoint of material cost. As concerns the present embodiment shown in Fig. 2, the absorbent core 4, being rectangular, has the same width at any position of measurement in its longitudinal direction. In case where the absorbent core 4 has its width varied in the longitudinal direction, for example, where it has the shape of a hourglass, the width W2 is measured at a position which corresponds to the position where the body-surrounding elastic members 71a and 71b are disposed.

Prevention of the absorbent core's bunching is also advantageous in that the outer appearance of the diaper 1 while worn is improved.

To prevent bunching of the absorbent core 4 is especially effective when the absorbent core 4 is thin, light or highly soft. In detail, (i) when the absorbent core 4 has a thickness of about 0.5 to 10 mm, particularly about 0.5 to 5 mm, or (ii) when it has a pulp fiber content of about 0 to 55% by weight, particularly about 0 to 30% by weight, or (iii) when it has a Taber stiffness, a measure of softness, of 4,90 to 196,23 mN·cm (0.5 to 20 gf·cm), particularly 4,90 to 98,07 mN·cm (0.5 to 10 gf·cm), in particular 4,90 to 49,03 mN·cm (0.5 to 5 gf·cm), the absorbent core 4 has a particularly good fit but is, in turn, liable to bunching. By arranging the body-surrounding elastic members 71a and 71b in the above-described configuration, such an absorbent core 4, although it is thin and light and therefore easily deformed, can effectively be prevented from bunching. Incidentally, where the absorbent core 4 has a pulp fiber content of 0 to 55% by weight, it is preferred for the absorbent core 4 to have a superabsorbent polymer content of 40 to 100% by weight, particularly 50 to 100% by weight. The absorbent core 4 can further contain other fibrous materials than pulp fiber, such as thermally bonding fiber.

The Taber stiffness can be determined in accordance with "Taber stiffness tester method for Paper and Board" specified in JIS P8125. Specifically, Taber stiffness of an absorbent core 4 (inclusive of enveloping tissue) cut into a size of 3.8 cm x 7.0 cm is measured with a Taber stiffness tester.

It is possible to use an absorbent core containing no pulp fiber. For example, an absorbent core comprising a nonwoven fabric web having superabsorbent polymer particles dispersed therein can be used. The proportion of the nonwoven fabric web is preferably 50 to 80% by weight, still preferably 60 to 70% by weight, and that of the superabsorbent polymer is preferably 20 to 60% by weight, still preferably 30 to 50% by weight.

In order to secure a good fit around the wearer's waist with a moderate tension, the ratio of the width (W3+W4) of parts of the body-surrounding elastic members 71a and 71b which manifest elastic contractibility as measured in their flat state to the width W1 of the diaper measured in its flat state is 40 to 95%, preferably 50 to 80%, still preferably 55 to 75%. The parts which manifest elastic contractibility are the parts where the body-surrounding elastic members are disposed in the body-surrounding side portions D1, i.e., the portions outside the side edges of the absorbent core 4. If the width ratio is smaller than 40%, the elastic members 71a and 71b must be fixed at a high elongation in order to provide a good fit. Such a high elongation results in unfavorable partial application of pressure to a wearer's body, giving similar inconveniences or discomfort to the wearer as with shorts type disposable diapers having contractible side panels. If the above width ratio exceeds 95%, on the other hand, the absorbent core 4 would be bunched or gathered to have reduced absorbing performance. Where the width of the body-surrounding elastic members 71a in the front portion A and that of the body-surrounding elastic members 71b in the rear portion B are different, at least one of the widths in the front and the rear portions A and B should be in the above-specified range. It is preferred that both widths in the front and the rear portions A and B be within the above range.

To ensure a good fit around the wearer with a moderate tension, it is desirable that the 30% elongation stress σ_{W} of the waist opening portion 5 be greater than the 30% elongation stress σ_{D} of the body-surrounding portion D in which the elastic members 71a and 71b are disposed. It is more desirable for developing a more proper tension around the wearer that σ_{W} be up to 10 times, particularly from 1.5 to 6 times, as great as σ_{D}.

For ease in putting the diaper 1 on a wearer, prevention of sagging and drooping of the diaper 1 while use, and alleviation of pressure imposed on the wearer's body, the waist opening portion 5 preferably has a 30% elongation stress σ_{w} of 29,42 to 245,12 N/m (30 to 250 gf/cm), particularly 49,03 to 147,10 N/m (50 to 150 gf/cm). For producing a moderate tension around the wearer's body, the body-surrounding portion D where the body-surrounding elastic members are disposed preferably has a 30% elongation stress σ_{D} of 4,90 to 98,07 N/m (5 to 100 gf/cm), particularly 9,81 to 39,23 N/m (10 to 40 gf/cm).

The disposable diaper 1 according to the present embodiment is preferably produced as follows. The absorbent body 10 is made in a usual manner. The exterior member 11 is prepared by applying a hot-melt self-adhesive on the entire surface of the outer sheet 12, fixing the body-surrounding elastic members 71a and 71b in their stretched state in the body-surrounding portion D along the width direction of the outer sheet 12, fixing the waist elastic members 51a and 51b and the leg elastic members 61a and 61b in their stretched state at the stated positions, superposing the inner sheet 13 on the outer sheet 12, and cutting the elastic members 71a and 71b at their center position in the diaper width direction. The absorbent body 10 and the exterior member 11 are partially bonded together. The side edges A1 and A2 of the exterior member 11 in the front portion A and the side edges B1 and B2 in the rear portion B are joined together, respectively, to make up the form of shorts.

In cutting the elastic members disposed in the body-surrounding portion D various known cutting means can be used, such as those composed of a cutting blade and a stationary blade, e.g., a rotary die cutter, a shear cutter and pinch-off blades, heat seal cutting, ultrasonic seal cutting, pressure cutting, water jet cutting, and so forth.

The present invention is not limited to the above-described embodiment, and various modifications can be made therein. For example, the body-surrounding elastic members 71a and 71b do not always need to have contractibility over the whole area of the portions D1 where they are disposed.

It is not always necessary that the body-surrounding elastic members 71a and 71b should be disposed on both the front portion A and the rear portion B. Where the elastic members are provided on only the front portion A, the diaper's appearance from the front is neat. Where the elastic members are provided on only the rear portion B, attachment of a tape strip for disposal of the diaper is easier, and printing, if made, is clearer.

While in the above embodiment the body-surrounding elastic members 71a and 71b are provided on side portions outside the absorbent core 4, it is possible to dispose at least one of them between the waist opening portion and the front and/or rear end edge of the absorbent core 4 in addition to the side portions, i.e., over the whole width of the diaper 1, provided that the absorbent core 4 is made shorter to leave enough space between their end edge(s) and the waist opening. The elastic member(s) located between the waist opening portion and the front and/or rear end edge may be contractible. Or alternatively, the elastic member(s) located between the waist opening portion and the front and/or rear end edge may be non-contractible at the region between two hypothetical lines which are extensions from the lateral side edges of the absorbent core 4, and may be contractible at the regions outside the two hypothetical lines.

In the above embodiment, which is one of the most preferred embodiments, the region of the body-surrounding elastic members 71a, 71b where the body-surrounding elastic members 71a, 71b overlap the lateral side edges of the absorbent core 4 is made non-contractible from the viewpoint of effectively obtaining the advantages of the present invention. The body-surrounding elastic members 71a, 71b may have, however, a contractible region in the area where the absorbent core 4 exists, provided that at least part of the elastic members 71a, 71b located in the area where the absorbent core 4 exists has a non-contractible region. The non-contractible region in the absorbent core 4 area is preferably at least about a half of the width W2 of the absorbent core 4 or between about 1/20 to 3/4 of the width W1 of the diaper 1 for use in shorts type diapers for babies; or preferably at least about 1/3 of the width W2 or between about 1/20 and 3/4 of the width W1 for use in shorts type diapers for incontinent adults.

The present invention will now be illustrated in greater detail with reference to Examples. The Examples are presented as being exemplary of the present invention and should not be construed as limiting.

### EXAMPLES 1 TO 4 AND COMPARATIVE EXAMPLES 1 AND 2

Shorts type disposable diapers shown in Figs. 1 and 2 were produced. The diaper width W1, the absorbent core width W2, and the body-surrounding elastic members width W3+W4 (width of the contractible parts of the body-surrounding elastic members) are shown in Table 1 below. Table 1 also includes the 30% elongation stress σ_{w} of the waist opening portion, the 30% elongation stress σ_{D} of the body-surrounding portion, and the Taber stiffness of the absorbent core. The diapers of Examples 1 to 3 and Comparative Examples 1 and 2 are for babies, and the diaper of Example 4 is for incontinent adults.

The 30% elongation stress σ_{W} of the waist opening portion and the 30% elongation stress σ_{D} of the body-surrounding portion of the diapers in Examples and Comparative Examples were measured as follows.

The waist opening portion of a diaper was cut 25 mm wide of the edge in the circumferential direction to make a ring specimen. Similarly, the body-surrounding portion of a diaper was cut in the circumferential direction to make a 70 mm wide ring specimen. The specimen was set between chucks of a tensile tester and pulled at a rate of 300 mm/min to measure the 30% elongation stress, which was divided by the width (cm) of the specimen to obtain the 30% elongation stress σ_{W} of the waist opening portion and the 30% elongation stress σ_{D} of the body-surrounding portion. The distance between the chucks was changed according to the width of a diaper. Where a diaper had the elastic members disposed only in one of the front portion and the rear portion, the specimen for measurement of σ_{D} was cut only out of the front or rear portion having the elastic members.

The diapers prepared in Examples and Comparative Examples were evaluated for fit, ease in putting on a wearer, and resistance to sagging in accordance with the following methods.

### 1) Fit

The diaper was put on 10 wearers, and the fit was judged from gapping between the wearer's body and the diaper and pressure application to the wearer's body and graded A to C. The wearers were ten people in which the wearers of the diapers of Examples 1 to 3 and Comparative Examples 1 and 2 were 16 to 24-month-old babies, and the wearers of the diaper of Example 4 were adults.
A Little gapping is observed. After the diaper is removed, no pressure marks by the elastic members are left on the body.
B Gaps are formed. After the diaper is removed, no pressure marks by the elastic members are left on the body.
C Gaps are formed. After the diaper is removed, pressure marks by the elastic members are left on the body.

### 2) Ease in putting diaper on wearer

Ease in putting the diaper on a wearer was graded A to C for ease of widening the diaper and ease of pulling up the diaper. The wearers were the same as in (1) above.
A The diaper is easy to widen in the part from the waist opening to the body-surrounding portion and to pull up.
B The diaper is hard to widen in the part from the waist opening to the body-surrounding portion.
C The diaper is hard to widen in the part from the waist opening to the body-surrounding portion and to pull up.

### 3) Resistance to sagging (sustained fit)

The diaper was put on a movable model, and the model was made to take a walking movement at a rate of 140 steps/min. Resistance of the diaper against sagging was judged from the slip (cm) of the diaper and graded A to C.
A The slip is less than 2 cm both for babies and for adults.
B The slip is 2 cm or more and less than 4 cm both for babies and for adults.
C The slip is 4 cm or more both for babies and for adults.

**TABLE 1**

| | Example | | | | Comparative Ex. | |
|---|---|---|---|---|---|---|
| | 1 | 2 | 3 | 4 | 1 | 2 |
| Diaper Width W1 (mm) | 380 | 380 | 300 | 600 | 380 | 300 |
| Absorbent Core Width W2 (mm) | 140 | 180 | 100 | 250 | 140 | 200 |
| Body-surrounding Elastic Member Width W3+W4 (mm) | 240 | 240 | 200 | 400 | 50 | 200 |
| Stress σ_{W} of Waist Opening | 98.07 | 98.07 | 78.46 | 176.52 | 98.07 | 78.46 |
| Portion (gf/cm) | (100) | (100) | (80) | (180) | (100) | (80) |
| Stress σ_{D} of Body-surrounding | 24.52 | 24.52 | 19.61 | 98.07 | 24.52 | 19.61 |
| Portion (gf/cm) | (25) | (25) | (20) | (100) | (25) | (20) |
| Taber Stiffness of Absorbent | 49.03 | 49.03 | 14.71 | 98.07 | 49.03 | 14.71 |
| Core (gf·cm) | (5) | (5) | (1.5) | (10) | (5) | (1.5) |
| Fit | A | A | A | B | C | B |
| Ease in Putting on Wearer | A | A | A | A | C | B |
| Resistance to Sagging | A | A | A | A | C | B |

It is seen from the results in Table 1 that the diapers of the present invention have a better fit and more ease in putting on a wearer, sustained fit to the wearer (hardly sags), and a satisfactory appearance while worn as compared with those of the Comparative Examples. The diaper of Comparative Example 1, having an insufficient elastic member width W3+W4 in the body-surrounding portion, has difficulty in pulling up and gives pressure to the wearer's body from the sides. Further, it forms gaps between the wearer's body and the wearer facing side of the diaper and sags considerably while use, which can cause leakage. The diaper of Comparative Example 2 is difficult to pull up and feels stiff because of its too wide absorbent core. Further, it forms gaps between the wearer's body and the wearer facing side thereof and tends to sag while worn, easily causing leaks.

As described above, the shorts type disposable diaper according to the present invention fits a large range of wearer sizes, is easy to put on a wearer, exerts moderate tension to provide a snug fit to a wearer, and has a neat appearance while worn. Further, the shorts type disposable diaper of the present invention realizes reduction of material cost while equalling conventional diapers in absorbing performance.

## Claims

1. A shorts type disposable diaper (1) comprising a liquid-permeable topsheet (2), a liquid-impermeable anti-leakage sheet (3) and a liquid-retentive absorbent core (4), and having a plurality of body-surrounding elastic members (71a, 71b) provided at a body-surrounding portion (D) which is located between a waist opening portion (5) and a leg opening portion (6), said body-surrounding elastic members (71a, 71b) extending in the circumferential direction of the diaper, **characterized in that**:
said body-surrounding elastic members (71a, 71b) are fixedly disposed in at least side portions which extend outward from longitudinal side edges of said absorbent core (4) in a stretched state so as to manifest elastic contractibility such that gathers are formed, but are not disposed in at least the center of the portion where said absorbent core (4) exists, or alternatively, are additionally disposed in the portion where said absorbent core (4) exists in such a manner that elastic contractibility does not manifest in at least said center,
the ratio of the width (W2) of said absorbent core at the position which corresponds to the position where said body-surrounding elastic members are disposed to the width (W1) of said diaper is 30 to 60%,
the ratio of the width (W3 + W4) of parts in which said elastic members manifest elastic contractibility to the width (W1) of said diaper is 40 to 95%, and
wherein said body-surrounding elastic members (71a, 71b) are disposed between an outer sheet which constitutes the outermost surface of said diaper and said anti-leakage sheet (3) or any other sheet.

2. The shorts type disposable diaper according to claim 1, wherein:
said diaper comprises an absorbent body (10) having said topsheet (2), said anti-leakage sheet (3) and said absorbent core (4) and an exterior member (11) having said outer sheet,
said exterior member (11) is disposed on the anti-leakage sheet side of said absorbent body (10), and
said absorbent body (10) and said exterior member are fixed together by partial bonding.

3. The shorts type disposable diaper according to claim 1 or 2, wherein:
an elastic member which extends in the circumferential direction of the diaper is fixedly disposed in the waist opening portion (5), and
the 30% elongation stress of said waist opening portion (5) is greater than the 30% elongation stress of the portion of said body-surrounding portion (D) where said body-surrounding elastic members are disposed.

4. The shorts type disposable diaper according to any of claims 1 to 3, wherein the 30% elongation stress of said waist opening portion (5) and said body surrounding portion (D) are from 29.42 to 245.12 N/m (30 to 250 gf/cm) and from 4.90 to 98.07 N/m (5 to 100 gf/cm), respectively.

5. The shorts type disposable diaper according to any of claims 1 to 4, wherein said absorbent core has a Taber stiffness of 4.90 to 196.13 mN·cm (0.5 to 20 gf·cm).

## Patentansprüche

1. Wegwerfwindel in Höschenform (1), die eine flüssigkeitsdurchlässige obere Schicht (2), eine flüssigkeitsundurchlässige Auslaufschutzschicht (3) und einen flüssigkeitshaltenden Absorptionskern (4) aufweist und mehrere körperumgebende elastische Elemente (71a, 71b) hat, die an einem körperumgebenden Teil (D) vorgesehen sind, der zwischen einem Taillenöffnungsteil (5) und einem Beinöffnungsteil (6) angeordnet ist, wobei die körperumgebenden elastischen Elemente (71a, 71b) sich in der Umfangsrichtung der Windel erstrecken, **dadurch gekennzeichnet, daß**
die körperumgebenden elastischen Elemente (71a, 71b) zumindest an Seitenteilen, die sich in einem gedehnten Zustand von den seitlichen Längsrändern des Absorptionskerns (4) nach außen erstrecken, um ein elastisches Zusammenziehungsvermögen zu zeigen, so daß Raffungen gebildet werden, fest angeordnet sind, aber zumindest in der Mitte des Teils, in dem der Absorptionskern (4) vorhanden ist, nicht angeordnet sind, oder alternativ in dem Teil, in dem der Absorptionskern (4) vorhanden ist, in einer derartigen Weise zusätzlich angeordnet sind, daß das elastische Zusammenziehungsvermögen sich zumindest in der Mitte nicht zeigt,
das Verhältnis der Breite (W2) des Absorptionskerns an der Stelle, die der Position entspricht, an der die körperumgebenden elastischen Elemente angeordnet sind, zu der Breite (W1) der Windel 30 bis 60% ist,
das Verhältnis der Breite (W3 + W4) von Teilen, in denen die elastischen Elemente ein elastisches Zusammenziehungsvermögen zeigen, zu der Breite (W1) der Windel 40 bis 95% ist, und
wobei die körperumgebenden elastischen Elemente (71a, 71b) zwischen einer äußeren Schicht, welche die äußerste Oberfläche der Windel bildet, und der Auslaufschutzschicht (3) oder einer beliebigen anderen Schicht angeordnet sind.

2. Wegwerfwindel in Höschenform nach Anspruch 1, wobei:
die Windel einen absorbierenden Körper (10) mit der oberen Schicht (2), der Auslaufschutzschicht (3) und dem Absorptionskern (4) und ein äußeres Element (11) mit der äußeren Schicht aufweist, wobei das äußere Element (11) auf der Auslaufschutzschichtseite des absorbierenden Körpers (10) angeordnet ist, und
der absorbierende Körper (10) und das äußere Element durch Teilverbinden aneinander befestigt sind.

3. Wegwerfwindel in Höschenform nach Anspruch 1 oder 2, wobei:
ein elastisches Element, das sich in der Umfangsrichtung der Windel erstreckt, fest in dem Taillenöffnungsteil (5) angeordnet ist, und
die 30%-Dehnungsspannung des Taillenöffnungsteils (5) größer als die 30%-Dehnungsspannung des Teils des körperumgebenden Teils (D) ist, wo die körperumgebenden elastischen Teile angeordnet sind.

4. Wegwerfwindel in Höschenform nach einem der Ansprüche 1 bis 3, wobei die 30%-Dehnungsspannung des Taillenöffnungsteils (5) und des körperumgebenden Teils (D) jeweils zwischen 29,42 und 245,12 N/m (30 bis 250 gf/cm) und zwischen 4,90 und 98,07 N/m (5 bis 100 gf/cm) liegen.

5. Wegwerfwindel in Höschenform nach einem der Ansprüche 1 bis 4, wobei der Absorptionskern eine Taber-Steifigkeit von 4,90 bis 196,13 mN·cm (0,5 bis 20 gf-cm) hat.

## Revendications

1. Couche jetable de type culotte (1) comprenant une feuille supérieure perméable au liquide (2), une feuille antifuite imperméable au liquide (3) et un coeur absorbant retenant le liquide (4), et présentant une pluralité d'éléments élastiques entourant le corps (71a, 71b) prévus au niveau d'une partie entourant le corps (D) qui est placée entre une partie d'ouverture pour la taille (5) et une partie d'ouverture pour la jambe (6), lesdits éléments élastiques entourant le corps (71a, 71b) s'étendant dans la direction circonférentielle de la couche, **caractérisée en ce que** :
lesdits éléments élastiques entourant le corps (71a, 71b) sont disposés fixement dans au moins les parties latérales qui s'étendent vers l'extérieur depuis les côtés latéraux longitudinaux dudit coeur absorbant (4) dans un état étiré, de manière à manifester une contractilité élastique telle que des fronces sont formées, mais ne sont pas disposés dans au moins le centre de la partie dans laquelle ledit coeur absorbant (4) est présente, ou en variante, sont disposés en plus dans la partie dans laquelle ledit coeur absorbant (4) est présent d'une manière telle que la contractilité élastique ne se manifeste pas dans au moins ledit centre,
le rapport de la largeur (W2) dudit coeur absorbant, en la position qui correspond à la position dans laquelle lesdits éléments élastiques entourant le corps sont disposés, sur la largeur (W1) de ladite couche est de 30 à 60 %,
le rapport de la largeur (W3 + W4) des parties dans lesquelles lesdits éléments élastiques manifestent une contractilité élastique sur la largeur (W1) de ladite couche est de 40 à 95 %, et
dans laquelle lesdits éléments élastiques entourant le corps (71a, 71b) sont disposés entre une feuille externe qui constitue la surface la plus extérieure de ladite couche et ladite feuille anti-fuite (3) ou n'importe quelle autre feuille.

2. Couche jetable du type culotte selon la revendication 1, dans laquelle :
ladite couche comprend un corps absorbant (10) présentant une feuille supérieure (2), ladite feuille anti-fuite (3) et ledit coeur absorbant (4) et un élément extérieur (11) présentant ladite feuille extérieure,
ledit élément extérieur (11) est disposé sur le côté de la feuille anti-fuite dudit corps absorbant (10), et
ledit corps absorbant (10) et ledit élément extérieur sont fixés conjointement par collage partiel.

3. Couche jetable du type culotte selon la revendication 1 ou 2, dans laquelle :
un élément élastique qui s'étend dans la direction circonférentielle de la couche est disposé fixement dans la partie d'ouverture pour la taille (5), et
la contrainte d'allongement de 30 % de ladite partie d'ouverture pour la taille (5) est supérieure à la contrainte d'allongement de 30 % de la portion de ladite partie entourant le corps (D) dans laquelle sont disposés lesdits éléments élastiques entourant le corps.

4. Couche jetable du type culotte selon l'une quelconque des revendications 1 à 3, dans laquelle la contrainte d'allongement de 30 % de ladite partie d'ouverture pour la taille (5) et de ladite partie entourant le corps (D) sont de 29,42 à 245,12 N/m (30 à 250 gf/cm) et de 4,90 à 98,07 N/m (5 à 100 gf/cm), respectivement.

5. Couche jetable du type culotte selon l'une quelconque des revendications 1 à 4, dans laquelle ledit coeur absorbant présente une rigidité Taber de 4,90 à 196,13 mN·cm (0,5 à 20 gf·cm).
